(19) Europäisches Patentamt
European Patent Office
Office européen des brevets

(11) Numéro de publication: **0 060 195**
**B1**

# (12) FASCICULE DE BREVET EUROPEEN

(45) Date de publication du fascicule du brevet: **29.01.86**

(21) Numéro de dépôt: **82400378.4**

(22) Date de dépôt: **04.03.82**

(51) Int. Cl.$^4$: **A 61 K 7/00,** A 61 K 31/19 // C07C53/16, C07C53/18

## (54) Application des acides halogénoacétiques à dose homéopathique, en médecine humaine et vétérinaire et dans les produits d'hygiène corporelle.

(30) Priorité: **05.03.81 FR 8104434**

(43) Date de publication de la demande:
**15.09.82 Bulletin 82/37**

(45) Mention de la délivrance du brevet:
**29.01.86 Bulletin 86/05**

(84) Etats contractants désignés:
**AT BE CH DE GB IT LI LU NL SE**

(56) Documents cités:
**FR-A-1 061 239**
**FR-A-1 295 338**
**FR-M- 2 293**
**FR-M- 6 070**
**US-A-3 179 562**
**US-A-4 122 188**

**FORMULAIRE PHARMACEUTIQUE, 1965, VIGOT FRERES, PARIS (FR), "Acide trichloracétique", page 56**
**DICTIONNAIRE VIDAL, 1961, OFFICE DE VULGARISATION PHARMACEUTIQUE, PARIS (FR), "ATS", page 147**

(73) Titulaire: **Le Foll, Jean**
**90, rue des Hauts Pavés**
**F-44000 Nantes (FR)**

(72) Inventeur: **Le Foll, Jean**
**90, rue des Hauts Pavés**
**F-44000 Nantes (FR)**

(74) Mandataire: **Combe, André et al**
***CABINET BEAU DE LOMENIE* 55 rue d'Amsterdam**
**F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 060 195 B1

(56) Documents cités:

**CHEMICAL ABSTRACTS, vol. 56, no. 10, 14 mai 1962, colonne 11730b, COLUMBUS OHIO (US)**
**THE MERCK INDEX, 1976, 9e edition, MERCK & CO., (US), "Dichloroacetic acid", page 402**
**CHEMISCHES ZENTRALBLATT, vol. 135, no. 40, 30 septembre 1964, abrégé 1437, (DE), G. BIZARD et al. "Die Sekretion von Galle durch die Leber der Ratte. 5. Mitt. Wirkung von Monojodessigsäure", page 169**
**CHEMICAL ABSTRACTS, vol. 77, no. 7, 14 août 1972, page 15, abrégé 43087r, COLUMBUS OHIO (US), P. DAL PRA et al. "Effects of group-selective reagents on monoamine oxidase and cholinesterase in guinea pig ileum"**
**RECUEIL DES TRAVAUX CHIMIQUES DES PAYS-BAS, 1947, (NL), NEDERLANDSCHE CHEMISCHE VEREENIGING, E. GRYSZKIEWICZ-TROCHIMOWSKI et al. "Recherches sur les composés organiques fluorés dans la série aliphatique", pages 419-426**

**Le dossier contient des informations techniques présentées postérieurement au dépôt de la demande et ne figurant pas dans le présent fascicule.**

**Description**

L'invention concerne l'application à dose homéopathique en médecine humaine et vétérinaire et dans les produits d'hygiène corporelle, des acides halogénoacétiques de formule:

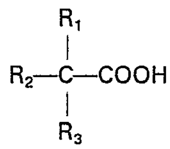

(I)

dans laquelle $R_1$, $R_2$, $R_3$ représentent chacun un àtome d'hydrogène ou d'halogène (F, Cl, Br, I), l'un au moins de ces radicaux représentant un atome d'halogène, et, lorsque deux ou trois d'entre eux représentent un halogène, ils peuvent être identiques ou différents.

Une des voies importantes du métabolisme cellulaire est celle qui est coiffée par le coenzyme A, coenzyme qui règle entre autres l'entrée de l'acétate dans le cycle de Krebs, la synthèse et la dégradation des lipides, la synthèse de certains acides aminés: alanine, valine, leucine, des phospho-amino-lipides, des médiateurs chimiques tels que l'acétylcholine.

L'ion acétate, ainsi que ses homologues supérieurs, peut difficilement intervenir seul dans le métabolisme par suite d'un phénomène de résonance:

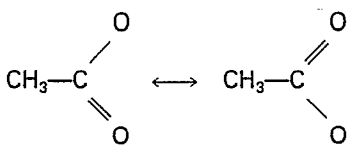

et doit étre activé par le CoA (travaux d'Ingraham-Green).

On a trouvé selon l'invention que, si l'on fait jouer un effet inductomère par un ou plusieurs halogènes: F—Cl—Br—I sur $CH_3$—COOH, on polarise la molécule qui, ainsi activée, se comporte, d'une part, comme le coenzyme A et, d'autre part, comme transporteur de l'atome ou des atomes qui ont modifié la résonance, lorsqu'on l'emploie à dose homéopathique.

Dans cette molécule nouvelle en biologie, l'effet inductomère peut être nuancé en jouant, d'une part, sur la nature des halogènes où les fractions de charge électroniques s'établissent comme suit: F=0,27, Cl=0,22, Br=0,20, I=0,16, et, d'autre part, sur leur répartition, permettant ainsi d'en moduler les effets thérapeutiques ou régulateurs du métabolisme.

L'expérimentation clinique des produits à dose homéopathique selon l'invention a montré que:

1) la substitution de 1, 2 ou 3 atomes d'hydrogène de l'acide acétique par 1, 2 ou 3 atomes de Cl permet d'obtenir une action générale bénéfique sur un organisme fatigué;

2) la substitution de 1, 2 ou 3 atomes d'hydrogène par 1, 2 ou 3 atomes de F oriente l'action thérapeutique sur les lésions osseuses en général, les lésions dentaires et les phenères (ongles, cheveux);

3) la substitution de 1, 2 ou 3 atomes d'hydrogène par 1, 2 ou 3 atomes de Br confère à la molécule une action protectrice particulière du système circulatoire capillaire;

4) la substitution de 1, 2 ou 3 atomes d'hydrogène par 1, 2 ou 3 atomes de I provoque une stimulation de la thyroïde et par là une régulation du métabolisme général, notamment au niveau de la peau, des phanères et des dystrophies dentaires.

L'invention a pour objet des compositions pharmaceutiques utiles en médecine humaine et vétérinaire et des produits d'hygiène corporelle, caractérisés en ce qu'ils contiennent un acide halogéno-acétique de formule I ci-dessus, à des dilutions comprises dans la gamme habituellement utilisée en homéopathie. Les compositions utilisables en médecine sont destinées soit à la voie orale et sont alors présentées sous forme de granules ou de solutions buvables préparés selon les procédés courants en homéopathie, soit à la voie parentérale sous forme de liquides injectables en ampoules, soit à la voie locale et sont préparés sous forme de lotions, crèmes, pommades, aérosols, collyres, suppositoires et huiles.

Dans les produits d'hygiène corporelle, les acides halogénoacétiques selon l'invention sont introduits à des dilutions homéopathiques dans les dentifrices, bains de bouche, shampooings, crèmes et huiles solaires.

On donne ci-après des exemples d'application des acides halogénoacétiques à dose homéopathique.

Exemple 1
Médecine vétérinaire

Un petit chien bâtard recueilli dans la rue 14 ans plus tôt se fait vieux et présente tous les signes d'une sénescence avancée: eczéma, poil rare, rugueux et râpeux du vieux chien. Il se traîne et a du mal à se déplacer.

On lui fait prendre matin et soir deux granules d'acide halogénoacétique en 5CH.

La semaine n'est pas terminée que l'eczéma a totalement disparu, le poil est redevenu lisse et soyeux et l'animal court.

Aujourd'hui, le chien a 18 ans passés et, lorsqu'il est fatigué, son maître lui administre avec le même succès le traitement qui l'avait sauvé quatre ans plus tôt.

Exemple 2
Médecine humaine

On rapporte la guérison d'une nécrose de la hanche en moins de quatre mois à la suite d'un traitement par la prise de trois granules d'acide halogénoacétique en 5CH, plus une dose en 30CH tous les huit jours.

Exemple 3

Un patient reçoit un fer à repasser brûlant (le câble est branché sur du 220 V) sur la paume de la

main droite. La douleur est intenable, des phlyctènes apparaissent.

Une application de la pommade suivante:

acide halogénoacétique en 4CH+5CH ââ 10%
excipient Q.S

calme la douleur en quelques instants et permet la cicatrisation de la peau en 24 h en même temps que le patient retrouve l'usage de sa main.

Exemple 4

De nombreuses expériences réalisées depuis 4 ans sur des personnes atteintes d'un coup de soleil ont montré que, même en cas de fortes brûlures, les sensations douloureuses disparaissent très rapidement sous l'effet d'un massage léger effectué avec une huile contenant:

acide halogénoacétique en 5CH+9CH+15CH

Exemple 5

Système capillaire

a) Alopécie

L'emploi d'un shampooing contenant la formule suivante arrête la chute des cheveux en un délai de 10 à 15 jours dans plus de 80% des cas:

acide halogénoacétique en 5CH
9CH ââ 10%
15CH
excipient Q.S.

en application 3 fois par semaine.

b) Calvitie

Lorsqu'il s'agit d'une calvitie, l'emploi bi-quotidien de la pommade suivante:

acide halogénoacétique en 5CH
7CH
9CH ââ 20%
15CH
excipient pour pommade Q.S.

permet de revivifier le cuir chevelu et l'on voit apparaître, au bout d'un certain temps variable suivant la personne, d'abord quelques cheveux fins, fragiles, qui tombent, se renouvellent en devenant de plus en plus forts et dont la densité en général est fonction de l'âge du sujet et de son tempérament.

Exemple 6

Odontostomatologie

La pathologie buccale est liée dans plus de 90% des cas à l'état de la denture et, par conséquent, à l'hygiène dentaire.

C'est peut-être ici que se manifestent de la façon la plus spectaculaire les qualités de cette formule halogénés telles qu'on les a définies. La formule générale:

acide halogénoacétique en 5CH
6CH ââ 20%
9CH
15CH
excipient Q.S.P. un tube de dentifrice,

donne des résultats spectaculaires.

a) Hygiène dentaire journalière

Le brossage régulier matin et soir après les repas, avec la formule selon l'invention, a permis d'établir sur un groupe de 50 personnes, par rapport à un groupe témoin qui employait un dentifrice courant, une fréquence moindre de 47,5% de caries au bout d'une année d'expérimentation.

b) Parodontolyses

Dans les cas de parodontolyses, un des symptômes de cette maladie, la congestion gingivale avec saignement au moindre contact, disparaît en un délai qui s'échelonne entre une et deux semaines, si l'on procède à un brossage après chaque repas.

De plus, dans de nombreux cas, au bout d'un temps variable, ou voit apparaître une amorce de régénérescence osseuse alvéolaire avec consolidation de la mobilité dentaire.

**Revendications**

1. Compositions pour remède homéopathique caractérisées en ce qu'elles contiennent l'un, au moins, des acides halogénoacétiques de formule:

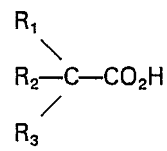

(dans laquelle $R_1$, $R_2$, $R_3$ représentent un atome d'hydrogène ou d'halogène, l'un au moins de ces radicaux représente un atome d'halogène et, quand deux ou trois d'entre eux représentent chacun un halogène, ils peuvent être identiques ou différents) à des dilutions comprises dans la gamme habituellement utilisée en homéopathie.

2. Compositions selon la revendication 1, caractérisées en ce qu'elles sont sous une forme administrable par voie orale telle que granulés ou solutions buvables.

3. Compositions selon la revendication 1, caractérisées en ce qu'elles sont sous une forme administrable par voie parentérale telle qu'ampoules injectables.

4. Compositions selon la revendication 1, caractérisées en ce qu'elles sont sous une forme administrable par voie locale telle que crèmes, pommades, lotions, aérosols, collyres, huiles.

5. Compositions d'hygiène corporelle, caractérisées en ce qu'elles contiennent l'un, au moins, des acides halogénoacétiques de formule:

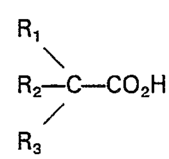

(dans laquelle $R_1$, $R_2$, $R_3$ représentent un atome d'hydrogène ou d'halogène, l'un au moins de ces radicaux représente un atome d'halogène et, quand deux ou trois d'entre eux représentent chacun un halogène, ils peuvent être identiques ou différents) à des dilutions supérieures ou égales à 4CH.

6. Compositions selon la revendication 5, caractérisées en ce qu'elle se présentent sous la forme de shampooings, dentifrices, bains de bouche, crèmes ou huiles solaires.

**Patentansprüche**

1. Zusammensetzungen für homöopathische Heilmittel, dadurch gekennzeichnet, daß sie mindestens eine der Halogenessigsäuren der Formel

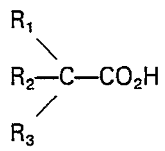

(worin $R_1$, $R_2$, $R_3$ ein Wasserstoff- oder Halogenatom darstellen, wobei mindestens eines dieser Radikale für ein Halogenatom steht und, wenn zwei oder drei davon jeweils für Halogen stehen, diese gleich oder verschieden sein können) in Verdünnungen enthalten, die im in der Homöopathie üblicherweise verwendeten Bereich liegen.

2. Zusammensetzungen nach Anspruch 1, dadurch gekennzeichnet, daß sie in auf oralem Weg verabreichbarer Form, wie Granülen oder Trinklösungen, vorliegen.

3. Zusammensetzungen nach Anspruch 1, dadurch gekennzeichnet, daß sie in auf parenteralem Weg verabreichbarer Form, wie Injektionsampullen, vorliegen.

4. Zusammensetzungen nach Anspruch 1, dadurch gekennzeichnet, daß sie in auf lokalem Weg verabreichbarer Form, wie Cremes, Pomaden, Lotionen, Aerosole, Salben, Öle, vorliegen.

5. Zusammensetzungen für Körperhygiene, dadurch gekennzeichnet, daß sie mindestens eine der Halogenessigsäuren der Formel

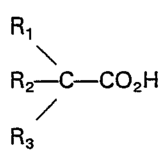

(worin $R_1$, $R_2$, $R_3$ ein Wasserstoff- oder Halogenatom darstellen, wobei mindestens eines dieser Radikale für ein Halogenatom steht und, wenn zwei oder drei davon jeweils für Halogen stehen, diese gleich oder verschieden sein können) in Verdünnungen von höher oder gleich 4CH enthalten.

6. Zusammensetzungen nach Anspruch 5, dadurch gekennzeichnet, daß sie in Form von Shampoos, Zahnpasten, Mundduschen, Sonnencremes oder -ölen, vorliegen.

**Claims**

1. Compositions for homeopathic remedy, characterized in that they contain at least one of the halogeno-acetic acids of formula:

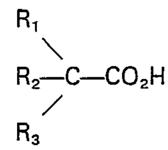

(in which $R_1$, $R_2$, $R_3$ are a hydrogen or halogen atom, at least one of the radicals is a halogen atom, and, when two or three among these radicals each are a halogen, they may be identical or different), at dilutions within the range normally used in homeopathy.

2. Compositions according to claim 1, characterized in that they are in a form to be administered by oral route such as granules or drinkable solutions.

3. Compositions according to claim 1, characterized in that they are in a form to be administered by parenteral route such as injectable ampoules.

4. Compositions according to claim 1, characterized in that they are in a form to be administered by local route such as creams, ointments, lotions, sprays, eye drops, oils.

5. Body health compositions, characterized in that they contain, at least one of the halogeno-acetic acids of formula:

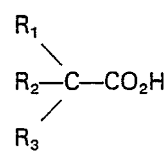

(in which $R_1$, $R_2$, $R_3$ are a hydrogen or halogen atom, at least one of these radicals is a halogen atom, and when two or three among these radicals each are a halogen, they may be identical or different) at dilutions higher than or equal to 4CH.

6. Compositions according to claim 5, characterized in that they are in the form of shampoos, toothpastes, mouth-washes, sun creams or oils.